# EUROPEAN PATENT APPLICATION

(11) **EP 1 892 515 A1**
(43) Date of publication of application: **27.02.2008**
(21) Application number: 06756883.2
(22) Date of filing: 01.06.2006
(51) Int. Cl.: G01N 1/10, G01N 33/48, G01N 33/50

(54) **METHOD OF COLLECTING BIOLOGICAL EXCRETORY SUBSTANCE**

(30) Priority: 03.06.2005 JP 2005164505
(71) Applicant: Nichirei Biosciences Inc., Chuo-ku Tokyo 104-8402 (JP)
(72) Inventor: MATSUSHITA, Hirohisa, Nichirei Biosciences Inc., Higashimurayama-shi, Tokyo 1890003 (JP); IKEMATSU, Hideyuki, Fukuoka-shi, Fukuoka 8130025 (JP)
(74) Representative: Uchida, Kenji
(86) International application number: PCT/JP2006/311006
(87) International publication number: WO 2006/129761

(57) **Abstract**

[Problems] An object of the present invention is to develop a sheet-shaped base material or a container having a structure which is designed in such a manner that snot or phlegm is collected as a biological excretory substance and said biological excretory substance is able to be easily applied to a test in medical therapy and also to develop a system where the biological excretory substance is collected using the same. [Solving Means] A biological excretory substance is collected using a bag-shaped container constituted from a sheet-shaped base material comprising a soft porous material only, a soft nonporous material only or a compounded material of a soft nonporous material and a soft porous material and then it is retained in the container.

## Description

### Technical Field

The present invention relates to an art concerning collection of a biological excretory substances and, more particularly, it relates to a total system where a biological excretory substances such as snot and phlegm are able to be collected and prepared as a medical test sample easily and at a low cost using a material in a sheet form having softness and good touch to the skin and being disposable.

The present invention also makes preparation of said sample possible by a less laborious method even for patients such as small children and also makes preparation of said sample possible by paying attention to a sense of shame and privacy of patients such as ladies.

### Background Art

With regard to a method for preparing a biological excretory substance such as snot and phlegm as a medical test sample, a method where said substance is compulsorily sucked by a pump using a catheter or the like has been carried out up to now.

However, as to a method where snot in the nasal cavity is compulsorily sucked by a pump using a catheter, there are following problems.
Thus, in conducting a method of collecting a test sample using a catheter, a tube of the catheter is inserted into a nasal cavity and, therefore, there are problems that has invasive property and also that a high cost is needed. According to said method, an economical burden of the patient becomes high.

Further, since there is no catheter which is exclusively used for suction of snot, the current status is to use a catheter for airway as a substitute therefor. Still further, since a catheter is to be inserted into the body, it is inevitable to be sterilized.

On the other hand, with regard to a tissue, studies for its raw material has been conducted with an object of mitigation of irritation and pain to the skin around the nose upon blowing one's nose and, although a patent application therefor has been filed already, there has been no report for the use of a tissue for collection of snot as a medical test sample.
Patent Document 1: JP-T-2003-526743
Non-Patent Document 1 : "Nanzando's Medical Dictionary" published on January 10, 1972 by Nanzando, page 217

### Disclosure of the Invention

### Problems that the Invention is to Solve

To develop a method for an efficient collection of a medical test sample such as phlegm and snot as a biological excretory substance easily and at a low cost in a mode of considering in a sense of shame and privacy of patients.

### Means for Solving the Problems

In order to solve the above-mentioned problems, the present inventors have investigated from various views and conducted various studies by carrying out approaches which are entirely different from the conventionally conducted methods. As a result, they have firstly found a method for collecting a medical test sample whereby the above-mentioned problems are able to be solved at a stroke by the use of a disposable sheet-shaped base material.

Thus, the basic idea of the present invention is a method for collecting a biological excretory substance characterized in using a sheet-shaped base material which is soft, has a good touch to the skin and is disposable (and is able to be burned if desired). The sheet-shaped base material may be used as it is to collect a biological excretory substance by, for example, blowing the nose or may be placed in a container to collect a biological excretory substance by, for example, blowing the nose near the opening of the container.

With regard to embodiments of the present invention, the following modes are covered for example.

### (Embodiment A)

A method of collecting a biological excretory substance using a sheet-shaped base material having softness and good touch to the skin and being disposable in which the sheet-shaped base material is used as it is or by folding or making into a bag, characterized in that,
the sheet-shaped base material is (a) that where a nonporous material is singularly used or plurally layered or (b) that where a porous material is singularly used or plurally layered or is (c) constituted from a compounded material in which nonporous and porous materials as such are layered.

### (Embodiment B)

The method according to the above (A), wherein a porous material which may be water repellent (such as tissue, filter paper, Japanese paper or nonwoven paper) is used in the inner side of a sheet-shaped base material while a nonporous material (such as plastic film or metal foil) is used in the outside and the biological excretory substance is collected in said inner side.

### (Embodiment C)

The method according to any of the above mentioned (A) to (B), wherein the biological excretory substance is snot, phlegm or saliva.

### (Embodiment D)

The method according to any of the above-mentioned (A) to (C), wherein the sheet-shaped base material is a tissue (such as a single or double pulp sheet in a size of (10 to 20 mm) × (15 to 30 mm); a commercially available product may be used as well).

### (Embodiment E)

A sheet-shaped base material which is used for conducting the method mentioned in any of the above (A) to (D).

### (Embodiment F)

The sheet-shaped base material according to the above-mentioned (E), wherein the sheet-shaped base material is used as it is, is used by folding or is used as a bag-shaped container after making into a bag.

In the present invention, a biological excretory substance is collected by blowing one's nose using a sheet-shaped base material as it is including tissue or the like or, in other words, by blowing the nose using a tissue in a normal way. Besides that, the present invention also covers an art in which a plastic film (film comprising at least one of polyvinyl chloride, polyethylene and polypropylene) is used as it is, a compounded material where tissue and plastic film are layered is used as it is or a commercially available bag for packing of food made of plastic film is used whereby a biological excretory substance is received therein. Incidentally, in the sheet-shaped base material, it is also possible that the materials of the same or different type are layered in two or more layers.

The present invention in case the biological excretory substance which is characterized in using a bag-shaped container includes, for example, the following embodiments.

### (Embodiment 1)

A method of collecting a biological excretory substance, characterized in that, a biological excretory substance is collected using a bag-shaped container comprising one or more soft nonporous material(s) only, comprising one or more soft porous material(s) only or being constituted from a sheet-shaped base material comprising a compounded material of one or more soft nonporous material (s) with one or more soft porous material(s) and then it is retained in the container.

### (Embodiment 2)

A sheet-shaped base material is prepared in such a manner that a porous material optionally having a water repellency appears outside (outer surface) while a nonporous material appears inside (inner surface) and adjacent two sides of those materials are adhered to prepare a bag-shaped container. In its use, surface and back are reversed by folding from a top to which the bag-shaped container is adhered to another top to which it is not adhered. As a result, a bag-shaped container (upon use) where the porous material is inside while the nonporous material is outside is completed. (If desired, the bag-shaped container may not be turned inside out but may be used as it is and, in that case, the order of the porous material and the nonporous material is reversed.) A method of collecting a biological excretory substance which is characterized in that a biological excretory substance is collected using the resulting bag-shaped container and retained in a container (a porous material surface).

### (Embodiment 3)

The method according to any of the above 1 or 2, wherein a bag-shaped container which is disposable and/or is able to be burned is used and the biological excretory substance is at least one substance selected from snot, phlegm and saliva.

### (Embodiment 4)

In a bag-shaped container used for carrying out the method mentioned in any of the above 1 to 3, a bag-shaped container which is characterized in that, in said bag-shaped container, a sheet-shaped base material constituting it comprises only a soft nonporous material or comprises a compounded material of a soft nonporous material with a soft porous material.

### (Embodiment 5)

The bag-shaped container according to the above 4, wherein the sheet-shaped base material comprising a nonporous material only or a sheet-shaped base material where two or more materials containing at least one nonporous material are layered is folded and at least one of the edges adjacent to the folded line is stuck together by at least one means selected from adhesion, fusion and pressurized adhesion.

### (Embodiment 6)

The bag-shaped container according to the above 4, wherein two or more sheets of materials containing at least one sheet of nonporous material are layered to prepare a sheet-shaped base material and, except for an opening, other edges are stuck together by at least one means selected from adhesion, fusion and pressurized adhesion so that the biological excretory substance does not leak outside.

### (Embodiment 7)

The bag-shaped container according to any of the above 4 to 6, wherein the nonporous material is a plastic film which is transparent, semitransparent or nontransparent or a metal foil (such as an aluminum foil) and the porous material is a soft material which does not absorb the liquid or has a low absorbing property.

### (Embodiment 8)

The bag-shaped container according to any of the above 4 to 7, wherein the shape (plane view) of the bag-shaped container is at least one of the shapes of triangle, tetragon, pentagon, hexagon, polygon, circle, ellipse and trapezoid.

### (Embodiment 9)

The bag-shaped container according to any of the above 4 to 8, wherein the container has such a strength that a self-standing property is available and has such a softness that a nose is able to be blown and the opening has such a size that one side is 5 to 8 cm or longer whereby a nose is able to be blown.

### (Embodiment 10)

The bag-shaped container according to any of the above 4 to 9, wherein a nonwoven paper sheet and/or tissue (if desired, such a porous material may be made water-repelling) are/is placed on the upper side of the tetragonal plastic sheet and the base material edges of the adjacent two sides are fused to make into a bag shape.

### (Embodiment 11)

The bag-shaped container according to any of the above 4 to 10, wherein, in actual use, the bag-shaped container is turned over and the biological excretory substance is placed inside and retained in the bottom while the container is opened and allowed to stand so that it is able to be immediately tested.

In the embodiments characterized by the above-mentioned bag-shaped container and also in the above-mentioned embodiments, the present invention further covers the following embodiments.

### (Embodiment a)

A sheet-shaped base material where letters (including data, record, etc.) are able to be written (including printing, rubber stamping, stamping treatment, etc.) on its outer surface is used or a thin piece (such as a paper label) in such a quality that the letters as mentioned above are able to be written is adhered on the outer side of the sheet-shaped base material.

### (Embodiment b)

If desired after the collection (and, if necessary, after the further retention) of the biological excretory substance, the opening is folded so that said base material is folded whereby the biological excretory substance is retained preventing the leakage of said substance outside or the adhesion of foreign matters from outside to said substance.
Further, after completion of the test, the biological excretory substance is able to be burned and/or disposed safety and easily together with said sheet-shaped base material.

### (Embodiment c)

A water-repelling nonwoven paper which is a nonwoven paper before addition of an ethylene gas absorber and is subjected to a water-repelling treatment is used as a porous material for said sheet-shaped base material.

### Advantages of the Invention

In accordance with the present invention, the nose is normally blown using a sheet-shaped base material and/or the nose is normally blown using a bag-shaped container whereby snot which is a medical test sample is able to be collected. The retained snot is subjected, for example, to a test for virus using a test kit, etc. and is diagnosed for influenza, etc. At that time, no big device such as catheter is necessary and a significant advantage that the operation is very simple accompanied with no pain is achieved. Moreover, in accordance with the present invention, a commercially available tissue such as a tissue for the face is used either as it is or by making into a container shape by folding into two or four and is able to be used as a sheet-shaped base material whereby the outcome is very good in terms of cost, easiness, etc. For example, a commercially available tissue of a double or single type with an indication of 197 mm length and 229 mm width according to the Household Good Quality Labeling Law of Japan is able to be used. The tissue as such may be subjected to a water-repelling processing.

Moreover, in conducting research and development of hay fever agents and antiallergic agents, snot of persons to be tested should be sampled and, for such an object, the sheet-shaped base material and the bag-shaped container according to the present invention are very effective. Further, when the inside (i.e., the side by which the nose is blown or the side to which nose, skin, etc. are contacted) is a porous material in the present invention, irritation to the skin is little and is soft whereby the nose is easily blown out and, still further, the collected snot is able to be retained just as it is if a water-repelling property is given thereto whereby even small amount of snot sample is well able to be utilized as a test sample.

Since the bag-shaped container according to the present invention is less expensive and is able to be made into disposable and burnable, the sterilizing treatment after the use for the repeated use is no longer necessary whereby the treatment after completion of the test is simple. Although it is of course possible that sterilization is previously conducted, there is a big advantage that a highly sterilizing treatment is not essential since the present container is not required to be placed into the body. Moreover, since collection of the test sample is possible by merely blowing the nose, the collection is not time-consuming and, in addition, the person to be tested himself/herself is able to collect the sample whereby, unlike in the case where catheter is used, there is no burden for medical doctors and nurses and the treatment after completion of the test is easy. Accordingly, the present invention is particularly good in the very busy medical job site. Since the collected excretory substance is not visible from outside, that results in protection of privacy of the test person and, as will be apparent from the Examples which will be mentioned later, the test sample collected by this container showed the same test result as in the case of the samples which were collected by the conventional devices.

### Best Mode for Carrying Out the Invention

The present invention relates to a method of collecting a biological excretory substance such as snot, phlegm and saliva and is characterized in using a sheet-shaped base material which may be disposable and/or relates to use of a bag-shaped container made into a bag shape using a sheet-shaped base material which may be disposable.

The sheet-shaped base material has softness and good touch to the skin and is disposable and said material is that where one or more sheet(s) of only nonporous material is/are used or layered, that where one or more sheet (s) of only porous material is/are used or layered or it is constituted from a compounded material in which one or more material(s) as such is/are used or layered. For such a purpose, for example, nonwoven paper having water repellency is commercially available and it may be used and, with regard to a porous material (such as paper, cloth and various fiber products), it may be used either as it is or may be treated with a water repellent. As to the water repellent, known ones may be appropriately used and examples thereof which are applicable are aluminum salt, wax dispersion which contains or does not contain zirconium, wax-like thermosetting resin, metal soap and metal salt, organometallic complex of chromium and aluminum, fluorinating chemicals and alkylketene dimer.

As mentioned above, the present invention relates to a method of collecting a biological excretory substance which is characterized in using a sheet-shaped base material or a bag-shaped container manufactured using the sheet-shaped base material. Although the collected biological excretory substance is preferred to be retained, the porous material is able to be used just as it is since a test sample for influenza, for example, is sufficient in such a small amount as 50 µl. However, with regard to the porous material, that having a water repellency is preferred than that having a hygroscopicity.

The present invention also provides a bag-shaped container in addition to the sheet-shaped base material used for carrying out the above-mentioned method. The bag-shaped container according to the present invention is prepared by making into a bag shape using a sheet-shaped base material comprising only a soft porous material or a soft nonporous material or using a sheet-shaped base material comprising a compounded material of nonporous and porous soft materials.

With regard to the bag-shaped container according to the present invention, that which is made into a bag shape where a layered sheet-shaped base material is used and, except an opening part, other edges are adhered using an adhesive, fused by a heating treatment or adhered by subjecting to a pressurized adhesion by means of fastening with zippers or that which is made into a bag using a sheet-shaped base material is appropriately used.

In accordance with the present invention, there is provided a bag-shaped container where a sheet-shaped base material solely comprising a porous material, a sheet-shaped base material solely comprising a nonporous material or a base material where two or more sheets of material containing at least one sheet of nonporous material (such as a laminated based material of nonporous material with porous material) is folded and at least one edge of the edges adjacent to the folded line is adhered as mentioned already. For the manufacture of the bag-shaped container, the above-mentioned sheet-shaped base material is used.

To be more specific, a rectangular sheet-shaped base material, for example, is folded into two along its central part and one edge being adjacent to the folded line is adhered to give a bag-shaped container where the bottom is closed in a triangular shape. Since its upper part opens in a triangular shape, it is easily able to blow out the nose and, since the snot is retained in the lower area, it is able to be used for the test.

In accordance with the present invention, there is further provided a bag-shaped container which is characterized in that two or more sheets of a material containing at least one sheet of nonporous material are layered to prepare a base material and, except an opening part, other edges are stuck by at least one selected from adhesion, fusion and pressurized adhesion means so that the biological excretory substance is not leaked outside.

In the present invention, any of transparent, semitransparent and nontransparent plastic film (film of polyvinyl chloride, polyethylene, polypropylene, etc.) or metal foil such as aluminum foil may be used as a nonporous material while, as a porous material, a soft material which absorbs liquid or absorbs little liquid (such as tissue, pulp paper, Japanese paper, filter paper and nonwoven paper) may be used. It may be further subjected to a water repelling treatment.

In the bag-shaped container of the present invention, its side upon use (after being assembled) is at least any of shapes of triangle, tetragon, pentagon, hexagon, polygon, circle, ellipse and trapezoid, has a strength of such an extent that it is able to stand without being supported and has a softness of such an extent that it is able to blow the nose and its opening has a size of such an extent that it is able to blow the nose. With regard to the base material for constituting the bag-shaped container, a sheet-shaped base material which is able to form the above-mentioned bag-shaped container is able to be appropriately used and, for example, a base material having a thickness which is similar to a tissue for the face and to a plastic bag for packing the food used in supermarket, etc. is able to be used.

Specific examples of the bag-shaped container according to the present invention are that where a nonwoven paper sheet is placed on the upper side of a hexagonal plastic sheet or two compounded sheets where both sheets are layered are laminated followed by fusing the base material edges of the adjacent two sides to make into a bag shape.

When the bag-shaped container according to the present invention is used, this is turned over and the opening is utilized for blowing the nose whereupon the snot is retained in the bottom of the bag-shaped container (or an excretory substance such as phlegm and saliva is placed in the bag-shaped container from an opening and retained in the bottom) . In the meanwhile, the bag-shaped container where the opening is opened is able to be laid on a stand or a rack. Therefore, the excretory substance (test sample) retained in the bottom is able to be immediately tested without opening the container.

With regard to the excretory substance, when a test for influenza is conducted using, for example, snot as a test sample, amount of 10 to 100 µl or, preferably, 40 to 60 µl such as 50 µl is sufficient. Thus, there is no necessity of collecting a large amount of the test sample and there is a very big advantage that burden to the person to be tested is small. In the case of an excretory substance other than snot or in the case of test for the thing other than influenza, the optimum collecting amount may be settled according to the detecting method by referring to the above-mentioned data.

Hereinabove, although an illustration was done by taking snot as an example of the excretory substance, it is also possible that other excretory substance is collected using the sheet-shaped base material or the bag-shaped container according to the present invention and, if necessary, it is retained. After completion of the test, the substance may be discarded together with said base material or said container and the after-treatment is easy. Since the present container is also able to be made disposable, the container after sterilization is able to be used and, after the test, it is able to be discarded whereby neither sterilization of the device nor washing and sterilization thereof after its use is necessary for each run whereby the present invention is quite significant.

### Examples

The present invention will now be more specifically illustrated by way of the following Examples although the present invention is not limited to those Examples only.

### (Example 1)

(1) Two 10 cm × 10 cm sheets prepared by thermal adhesion of a polyethylene material (Seisan Nipponsha Ltd. ; Unipack H-4) using a heat sealer with a water-repelling nonwoven paper (Rengo Co., Ltd.; Super Green Pack EG before addition of an ethylene gas absorber) were layered where the polyethylene material side was inside and its adjacent two sides were adhered by fusion to prepare a bag-shaped product. When the resulting bag was turned over, the polyethylene side became outside which prevents the staining by, for example, snot and the nonwoven paper inside makes the breaking of blowing the nose good. Moreover, as a result of the use of two types of materials, the snot in the container is not directly visible making the resistance of the collector little. The collected excretory substance was accumulated to the bottom whereby, in the test which will be conducted later, the handling is made easy.

(2) The size of the container may be appropriately changed and, for example, it may be made 5 to 15 cm × 5 to 15 cm. The shape may not be a square but may be a rectangle. For example, in collecting the phlegm, the size may be smaller than in the above-mentioned case for snot and it may be, for example, about 6 cm × 6 cm.

(3) Two sheets each being 8 cm × 8 cm prepared by adhesion of said polyethylene material with said water repelling nonwoven fabric by means of a heat sealer were layered making the water repelling nonwoven fabric inside (i.e., layered in a reversed manner as compared with the case of (1)) and the adjacent two sides were adhered by fusion to prepare a bag. In the practical use, it may not be turned over but may be used just as it is. When the porous material to which water repellency was given is used, the product is soft to the skin and the collected sample is not absorbed but is accumulated whereby there is achieved a significant advantage that the sample in a small amount will do.

### (Example 2)

From a snot test sample collected from a patient showing an influenza-like symptom, a trial sample was prepared using a catheter while another trial sample was prepared using the container manufactured in Example 1(1) and existence of influenza virus in each of the snot samples was checked and compared. Existence of influenza virus was carried out using a reagent which was already approved by the Ministry of Health, Labor and Welfare (Statmark Influenza A/B (Number of Approval: 21600AMZ0057100)). As a result, as compared with a liquid sucked from the nasal cavity which is expensive and has invasive property to the patient, it is possible in the case of the present container that the patient himself/herself is able to conduct the collection and there is almost no invasive property. In a diagnostic result for infection of influenza, there is no difference in the result between the liquid sucked from the nasal cavity and the snot collected by the present container. Accordingly, diagnostic and analytical methods by collection of snot using the present container were found to be better than the conventional method due to easiness, low cost and no invasive property (Table 1).

**(Table 1)**

| Samples | Liquid sucked from nasal cavity | Self-excreted snot |
|---|---|---|
| 1 | negative | negative |
| 2 | positive to influenza A type | positive to influenza A type |
| 3 | negative | negative |
| 4 | negative | negative |
| 5 | positive to influenza A type | positive to influenza A type |
| 6 | positive to influenza A type | positive to influenza A type |
| 7 | positive to influenza B type | positive to influenza B type |
| 8 | negative | negative |
| 9 | positive to influenza B type | positive to influenza B type |
| 10 | positive to influenza B type | positive to influenza B type |

## Claims

1. A method of collecting a biological excretory substance, **characterized in that**, a sheet-shaped base material which is soft, has a good touch to the skin and is disposable is used.

2. The method according to claim 1, wherein said sheet-shaped base material is used as it is or by folding or by making into a bag.

3. The method according to claim 1 or 2, wherein said sheet-shaped base material is that (a) singular nonporous material is used or plural ones are layered or (b) singular porous material is used or plural ones are layered or (c) it is composed of a compounded material where the nonporous material and porous material are layered.

4. The method according to any of claims 1 to 3, wherein a water repelling porous material is used in the inside of said sheet-shaped base material while a nonporous material is used in the outside thereof and the biological excretory substance is collected by said inside.

5. The method according to any of claims 1 to 4, wherein the biological excretory substance is snot, phlegm or saliva.

6. The method according to any of claims 1 to 5, wherein a material on which letters are able to be written is used outside said sheet-shaped base material or a thin piece of a material on which letters are able to be written is adhered to said outside.

7. The method according to any of claims 1 to 6, wherein said sheet-shaped base material is a tissue.

8. The method according to any of claims 1 to 7, wherein, after the biological excretory substance is collected, said base material is folded and said biological excretory substance is retained.

9. The method according to any of claims 1 to 8, wherein, after completion of the test, the biological excretory substance is able to be safely disposed together with said sheet-shaped base material.

10. The method according to any of claims 1 to 9, wherein a water-repelling nonwoven paper before addition of an ethylene gas absorber is used as a porous material for said sheet-shaped base material.

11. A sheet-shaped base material used for carrying out the method mentioned in any of claims 1 to 10.

12. The sheet-shaped base material according to claim 11, wherein the sheet-shaped base material is used as it is or is used as a bag-shaped container after making into a bag.

13. The sheet-shaped base material according to claim 12, wherein a water-repelling nonwoven paper before addition of an ethylene gas absorber is used as a nonporous material for said sheet-shaped base material.
